# EUROPEAN PATENT APPLICATION

(11) **EP 4 206 334 A1**
(43) Date of publication of application: **05.07.2023**
(21) Application number: 21862065.6
(22) Date of filing: 25.08.2021
(51) Int. Cl.: C12Q 1/6883

(54) **MICRO RNA BIOMARKER FOR PREDICTING DRUG RESPONSE TO ANTIDIABETICS AND USE THEREOF**

(30) Priority: 28.08.2020 KR 20200109390; 28.08.2020 KR 20200109391; 28.08.2020 KR 20200109392
(71) Applicant: Industry Academic Cooperation Foundation Keimyung University, Daegu 42601 (KR)
(72) Inventor: CHO, Ho Chan, Daegu 42187 (KR); BAE, Yun Ui, Daegu 42922 (KR); PARK, Jae Hyung, Daegu 42070 (KR)
(74) Representative: Dehns
(86) International application number: PCT/KR2021/011373
(87) International publication number: WO 2022/045768

(57) **Abstract**

The present invention relates to a microRNA biomarker for predicting drug responsiveness to antidiabetics, and a use thereof.

In the present invention, the drug responsiveness prediction markers miR-136-3p, miR-150-3p, miR-15a-3p, miR-337-3p, miR-369-3p, miR-376a-3p, miR-379-5p, miR-483-3p, miR-495-3p, and miR-6126 were selected by comparing miRNA profiles of a normal group and a diabetes patient group before and after administering an SGLT2 inhibitor that is an antidiabetic. Although it was confirmed that the expression patterns of the selected markers differed for the normal group and the diabetes patient group, it was confirmed that the expression patterns for the diabetes patient group were restored so as to be similar to those for the normal group when the SGLT2 inhibitor that is an antidiabetic was administered to the diabetes patient group. Therefore, the marker according to the present invention can be useful as a marker for predicting drug responsiveness to antidiabetics.

## Description

### [Technical Field]

The present application claims priority to and the benefit of Korean Patent Application Nos. 10-2020-0109390, 10-2020-0109391, and 10-2020-0109392 filed in the Korean Intellectual Property Office on August 28, 2020, the entire contents of which are reference documents of the present application.

The present invention relates to a microRNA biomarker for predicting drug responsiveness to antidiabetics and a use thereof, and more specifically, to a biomarker composition for predicting drug responsiveness to antidiabetics, including one or more selected from the group consisting of miR-136-3p, miR-150-3p, miR-15a-3p, miR-337-3p, miR-369-3p, miR-376a-3p, miR-379-5p, miR-483-3p, miR-495-3p and/or miR-6126, a composition for predicting drug responsiveness to antidiabetics, including an agent that measures the expression level of the biomarker, and a method for providing information on drug responsiveness to antidiabetics using the biomarker.

### [Background Art]

Diabetes (diabetes mellitus) is a metabolic disease characterized by hyperglycemia occurring due to defects in insulin secretion or insulin action, and is a chronic degenerative disease that occurs due to insufficient insulin or reduced biological effects of insulin on target cells and is accompanied by complications such as renal failure due to its long duration.

In particular, type 2 diabetes occurs when insulin secretion is reduced due to acquired factors such as lack of exercise, obesity, or stress and/or blood sugar regulation fails due to the failure of insulin to perform its function.

Currently, the prevalence of diabetes is increasing year by year due to changes in dietary habits accompanied by rapid economic development, and thus, has reached about 5 to 10% in Korea, and according to statistics in 2005, the number of patients with diabetes exceeded 4 million, accounting for 8.3 out of 100 people, and is estimated to increase to about 6.8 million by 2025 (Korea Diabetes Information Center, 2005). Diabetes is the third most serious disease in the world and the fourth leading cause of death in Korea, and shortens life expectancy by 5 to 10 years due to various complications.

Currently known therapeutic agents for type 2 diabetes may be classified into four major types: sulfonylurea-based drugs that induce insulin secretion; biguanide-based drugs that transfer glucose to muscle cells and suppress glucose synthesis in the liver; alpha-glucosidase (α-glucosidase) inhibitors that suppress the enzyme that produces glucose in the small intestine; and thiazolidinedione (TZD)-based drugs that activate peroxisome proliferator-activated receptors (PPAR)-γ associated with adipocyte differentiation (Amir Babiker and Mohammed Al Dubayee et al., Sudan J Paediatr, 17(2): 11-20, 2017). Recently, sodium glucose cotransporter 2 (SGLT2) inhibitors regulate blood sugar by the method of suppressing the reabsorption of glucose in the kidneys to excrete glucose into the urine, and have the advantage of being able to be used in combination with other diabetic drugs because their mechanisms of action do not overlap those of other diabetic drugs.

However, such oral hypoglycemic agent drugs have a problem of many accompanying side effects such as hypoglycemic induction (sulfonylurea-based drugs), nephrotoxicity (biguanide-based drugs), lactic acidosis (biguanide-based drugs), diarrhea and upset stomach (alpha-glucosidase inhibitors) and weight loss.

### [Disclosure]

### [Technical Problem]

In the present invention, as a result of intensive studies to select a biomarker for predicting drug responsiveness to antidiabetics such that the side effects of drugs can be reduced by predicting drug responsiveness to antidiabetics and diabetes can be treated by selecting drugs having excellent effects, miR-136-3p, miR-150-3p, miR-15a-3p, miR-337-3p, miR-369-3p, miR-376a-3p, miR-379-5p, miR-483-3p, miR-495-3p and/or miR-6126 markers were selected. Although it was confirmed that the expression patterns of these markers differed between the normal group and the diabetes patient group, it was confirmed that the expression patterns for the diabetes patient group were restored so as to be similar to those for the normal group when an SGLT2 inhibitor, which is an antidiabetic, was administered to the diabetes patient group, thereby completing the present invention.

An object of the present invention is to provide a biomarker for predicting drug responsiveness to antidiabetics.

Another object of the present invention is to provide a composition for predicting drug responsiveness to antidiabetics, including an agent that measures the expression level of a biomarker and a method for providing information on drug responsiveness to antidiabetics using the biomarker.

Still another object of the present invention is to provide a method for screening antidiabetics using the biomarker.

### [Technical Solution]

To achieve the above-described objects,
the present invention provides a biomarker composition for predicting drug responsiveness to antidiabetics including one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126.

In a preferred embodiment of the present invention, the miR-136-3p may be represented by a base sequence of SEQ ID NO: 1, the miR-150-3p may be represented by a base sequence of SEQ ID NO: 2, the miR-15a-3p may be represented by a base sequence of SEQ ID NO: 3, the miR-337-3p may be represented by a base sequence of SEQ ID NO: 4, the miR-369-3p may be represented by a base sequence of SEQ ID NO: 5, the miR-376a-3p may be represented by a base sequence of SEQ ID NO: 6, the miR-379-5p may be represented by a base sequence of SEQ ID NO: 7, the miR-483-3p may be represented by a base sequence of SEQ ID NO: 8, the miR-495-3p may be represented by a base sequence of SEQ ID NO: 9, and the miR-6126 may be represented by a base sequence of SEQ ID NO: 10.

In another preferred embodiment of the present invention, the antidiabetic may be a sodium glucose cotransporter 2 (SGLT2) inhibitor.

In still another preferred embodiment of the present invention, the diabetes may be type 2 diabetes.

To achieve other objects,
the present invention provides a composition for predicting drug responsiveness to antidiabetics, including an agent that measures the expression levels of one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126.

In a preferred embodiment of the present invention, the miR-136-3p may be represented by a base sequence of SEQ ID NO: 1, the miR-150-3p may be represented by a base sequence of SEQ ID NO: 2, the miR-15a-3p may be represented by a base sequence of SEQ ID NO: 3, the miR-337-3p may be represented by a base sequence of SEQ ID NO: 4, the miR-369-3p may be represented by a base sequence of SEQ ID NO: 5, the miR-376a-3p may be represented by a base sequence of SEQ ID NO: 6, the miR-379-5p may be represented by a base sequence of SEQ ID NO: 7, the miR-483-3p may be represented by a base sequence of SEQ ID NO: 8, the miR-495-3p may be represented by a base sequence of SEQ ID NO: 9, and the miR-6126 may be represented by a base sequence of SEQ ID NO: 10.

In another preferred embodiment of the present invention, the antidiabetic may be a sodium glucose cotransporter 2 (SGLT2) inhibitor.

In still another preferred embodiment of the present invention, the agent that measures the expression level may be a sense and antisense primer or probe, which complementarily binds to microRNA.

Further, the present invention provides a diagnostic kit for predicting drug responsiveness to antidiabetics, including the composition for predicting drug responsiveness to antidiabetics.

In addition, the present invention provides a method for providing information on drug responsiveness to an antidiabetic, the method including: (a) measuring the expression levels of one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126 in a biological sample derived from a subject before and after the administration of the antidiabetic; and (b) comparing the microRNA expression levels before and after the administration of the antidiabetic.

In a preferred embodiment of the present invention, the miR-136-3p may be represented by a base sequence of SEQ ID NO: 1, the miR-150-3p may be represented by a base sequence of SEQ ID NO: 2, the miR-15a-3p may be represented by a base sequence of SEQ ID NO: 3, the miR-337-3p may be represented by a base sequence of SEQ ID NO: 4, the miR-369-3p may be represented by a base sequence of SEQ ID NO: 5, the miR-376a-3p may be represented by a base sequence of SEQ ID NO: 6, the miR-379-5p may be represented by a base sequence of SEQ ID NO: 7, the miR-483-3p may be represented by a base sequence of SEQ ID NO: 8, the miR-495-3p may be represented by a base sequence of SEQ ID NO: 9, and the miR-6126 may be represented by a base sequence of SEQ ID NO: 10.

In another preferred embodiment of the present invention, the agent that measures the expression level may be a sense and antisense primer or probe, which complementarily binds to microRNA.

In still another preferred embodiment of the present invention, the expression level may be measured through one or more methods selected from the group consisting of next generation sequencing (NGS), polymerization chain reaction (PCR), reverse transcription-polymerase chain reaction (RT-PCR), real-time polymerization chain reaction (PCR), an RNase protection assay (RPA), a microarray, and northern blotting.

In yet another preferred embodiment of the present invention, the antidiabetic may be a sodium glucose cotransporter 2 (SGLT2) inhibitor.

In a preferred embodiment of the present invention, after the administration of the antidiabetic, when the miR-136-3p, miR-15a-3p, miR-337-3p, miR-369-3p, miR-376a-3p, miR-379-5p or miR-495-3p expression is increased or the miR-150-3p, miR-483-3p or miR-6126 expression is decreased, information may be provided that the antidiabetic has a therapeutic effect.

The present invention, in an aspect, relates to a biomarker composition for predicting drug responsiveness to antidiabetics, including one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126.

In the present invention, the diabetes may be type 2 diabetes.

In the present invention, the miR-136-3p may be represented by the base sequence of SEQ ID NO: 1, and may include a base sequence having a sequence homology of 70% or more, preferably 80% or more, more preferably 90% or more, and most preferably 95% or more with the base sequence represented by SEQ ID NO: 1.

In the present invention, the miR-150-3p may be represented by the base sequence of SEQ ID NO: 2, and may include a base sequence having a sequence homology of 70% or more, preferably 80% or more, more preferably 90% or more, and most preferably 95% or more with the base sequence represented by SEQ ID NO: 2.

In the present invention, the miR-15a-3p may be represented by the base sequence of SEQ ID NO: 3, and may include a base sequence having a sequence homology of 70% or more, preferably 80% or more, more preferably 90% or more, and most preferably 95% or more with the base sequence represented by SEQ ID NO: 3.

In the present invention, the miR-337-3p may be represented by the base sequence of SEQ ID NO: 4, and may include a base sequence having a sequence homology of 70% or more, preferably 80% or more, more preferably 90% or more, and most preferably 95% or more with the base sequence represented by SEQ ID NO: 4.

In the present invention, the miR-369-3p may be represented by the base sequence of SEQ ID NO: 5, and may include a base sequence having a sequence homology of 70% or more, preferably 80% or more, more preferably 90% or more, and most preferably 95% or more with the base sequence represented by SEQ ID NO: 5.

In the present invention, the miR-376a-3p may be represented by the base sequence of SEQ ID NO: 6, and may include a base sequence having a sequence homology of 70% or more, preferably 80% or more, more preferably 90% or more, and most preferably 95% or more with the base sequence represented by SEQ ID NO: 6.

In the present invention, the miR-379-5p may be represented by the base sequence of SEQ ID NO: 7, and may include a base sequence having a sequence homology of 70% or more, preferably 80% or more, more preferably 90% or more, and most preferably 95% or more with the base sequence represented by SEQ ID NO: 7.

In the present invention, the miR-483-3p may be represented by the base sequence of SEQ ID NO: 8, and may include a base sequence having a sequence homology of 70% or more, preferably 80% or more, more preferably 90% or more, and most preferably 95% or more with the base sequence represented by SEQ ID NO: 8.

In the present invention, the miR-495-3p may be represented by the base sequence of SEQ ID NO: 9, and may include a base sequence having a sequence homology of 70% or more, preferably 80% or more, more preferably 90% or more, and most preferably 95% or more with the base sequence represented by SEQ ID NO: 9.

In the present invention, the miR-6126 may be represented by the base sequence of SEQ ID NO: 10, and may include a base sequence having a sequence homology of 70% or more, preferably 80% or more, more preferably 90% or more, and most preferably 95% or more with the base sequence represented by SEQ ID NO: 10.

In the present invention, the antidiabetic may be a sodium glucose cotransporter 2 (SGLT2) inhibitor.

In a specific exemplary embodiment of the present invention, it was intended to select a marker for predicting drug responsiveness to antidiabetics, an SGLT2 inhibitor was used as an antidiabetic, and the expression level of microRNA in exosomes isolated from each serum of a normal group, a diabetes patient group before taking the SGLT2 inhibitor, and the diabetes patient group after taking the SGLT2 inhibitor was confirmed.

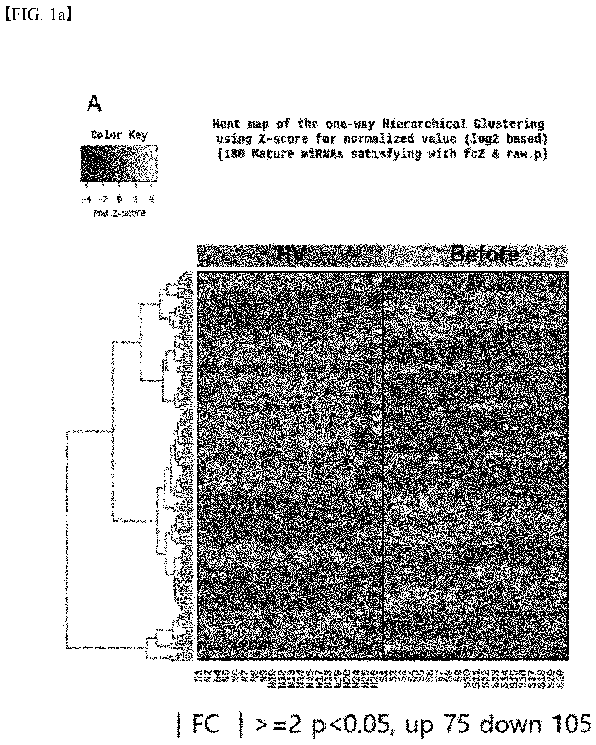

To select a microRNA marker for predicting drug responsiveness, as illustrated in FIGS. 1A to 3B, after dividing subjects to be analyzed into a normal group and a diabetes patient group before taking the SGLT2 inhibitor; a diabetes patient group before taking the SGLT2 inhibitor and a diabetes patient group after taking the SGLT2 inhibitor; and a normal group and a diabetes patient group after taking the SGLT2 inhibitor, respectively, the expression levels of microRNAs whose expression was significantly decreased or increased by 2-fold or more were visualized with a heatmap and analyzed by a volcano plot.

As a result, as shown in Table 2, microRNAs whose expression levels differed between the normal group and diabetes patients before taking the SGLT2 inhibitor, and whose expression patterns were restored so as to be similar to those of the normal group by taking the SGLT2 inhibitor were finally selected.

As a result of analyzing the expression levels of selected miR-136-3p, miR-150-3p, miR-15a-3p, miR-337-3p, miR-369-3p, miR-376a-3p, miR-379-5p, miR-483-3p, miR-495-3p and miR-6126 after dividing subjects to be analyzed into a normal group (HV), a diabetes patient group before taking the SGLT2 inhibitor (Before) and a diabetes patient group after taking the inhibitor (After), it was confirmed that a remarkable difference in expression levels of all of the miR-136-3p, miR-150-3p, miR-15a-3p, miR-337-3p, miR-369-3p, miR-376a-3p, miR-379-5p, miR-483-3p, miR-495-3p and miR-6126 was observed between the normal group and the diabetes patient group before taking the antidiabetic, and the expression levels were restored so as to be similar to those for the normal group by taking the SGLT2 inhibitor.

Therefore, the markers selected in the present invention may be used for markers for predicting drug responsiveness to antidiabetics, compositions for predicting drug responsiveness to antidiabetics, and methods for providing information on drug responsiveness to antidiabetics.

Furthermore, since it was confirmed that the microRNA marker of the present invention exhibits a remarkable difference in expression level between the normal group and the diabetes patient group, the microRNA marker may be used not only as a marker for predicting drug responsiveness but also as a diagnostic marker for diabetes.

The present invention, in another aspect, relates to a composition for predicting drug responsiveness to antidiabetics, including an agent that measures the expression levels of one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126.

The composition for predicting drug responsiveness to antidiabetics is used to confirm the expression level of the marker for predicting drug responsiveness to antidiabetics according to the present invention, and the specific details thereof are as described above.

In the present invention, the agent that measures the expression level may be a primer pair or probe, which complementarily binds to microRNA.

As used herein, the term "primer" refers to a primer pair which includes a forward and reverse primer pair, but preferably provides analytical results having specificity and sensitivity, as a fragment that recognizes a target gene sequence.

As used herein, the term "probe" refers to a material capable of specifically binding to a target material to be detected in a sample, and refers to a material capable of specifically confirming the presence of a target material in a sample through the binding. The type of probe is not limited to materials typically used in the art, but may be preferably a peptide nucleic acid (PNA), a locked nucleic acid (LNA), a peptide, a polypeptide, a protein, RNA or DNA, and is most preferably PNA.

The present invention, in still another aspect, relates to a diagnostic kit for predicting drug responsiveness to antidiabetics, including the composition for predicting drug responsiveness to antidiabetics.

The kit may be prepared by a typical preparation method known in the art. The kit may include, for example, an antibody in a lyophilized form and a buffer, a stabilizer, an inert protein, and the like.

The kit may further include a detectable label. The term "detectable label" refers to an atom or molecule that allows a molecule including a label among the same type of label-free molecules to be specifically detected. The detectable label may be attached to an antibody, interacting protein, ligand, nanoparticle, or aptamer, which specifically binds to the protein or fragment thereof. The detectable label may include a radionuclide, a fluorophore, and an enzyme.

The kit may be used according to a method such as next generation sequencing (NGS), polymerase chain reaction (PCR), reverse transcription-polymerase chain reaction (RT-PCR), real-time polymerase chain reaction (PCR), an RNase protection assay (RPA), and a microarray in order to analyze microRNA expression levels.

For example, the kit of the present invention may be a kit including a primer set specific to the marker gene of the present invention, an appropriate amount of DNA polymerase, a dNTP mixture, a PCR buffer solution and water in order to perform PCR. The PCR buffer solution may contain KCl, Tris-HCl and MgCl₂. In addition, components necessary for performing electrophoresis capable of confirming whether or not the PCR product is amplified may be additionally included in the kit of the present invention.

Furthermore, the kit of the present invention may be a kit including essential elements necessary for performing RT-PCR. The RT-PCR kit may include not only each primer pair specific for a marker gene, but also a test tube or another appropriate container, a reaction buffer, deoxynucleotides (dNTPs), enzymes such as Taq-polymerase and reverse transcriptase, DNase, a RNase suppressor, DEPC-water, sterilized water, and the like. Further, the RT-PCR may include a primer pair specific for a gene used as a quantitative control.

The present invention, in yet another aspect, relates to a method for providing information on drug responsiveness to an antidiabetic, the method including: (a) measuring the expression levels of one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126 in a biological sample derived from a subject before and after the administration of the antidiabetic; and
(b) comparing the microRNA expression levels before and after the administration of the antidiabetic.

In the method, the "biological sample" refers to a sample such as tissue, cells, blood, serum, plasma, saliva, cerebrospinal fluid, or urine, which has different microRNA expression levels due to the onset of diabetes, and preferably blood, plasma or serum, and more preferably exosomes included in serum.

The expression level in Step (a) may be measured through one or more methods selected from the group consisting of next generation sequencing (NGS), polymerization chain reaction (PCR), reverse transcription-polymerase chain reaction (RT-PCR), real-time polymerization chain reaction (PCR), an RNase protection assay (RPA), a microarray, and northern blotting.

In the present invention, after the administration of the antidiabetic, when the miR-136-3p, miR-15a-3p, miR-337-3p, miR-369-3p, miR-376a-3p, miR-379-5p or miR-495-3p expression is increased or the miR-150-3p, miR-483-3p or miR-6126 expression is decreased, drug responsiveness to the antidiabetic is excellent, so that it is possible to provide information that the antidiabetic is effective for treating diabetes. Preferably, when an SGLT2 inhibitor is administered as an antidiabetic, it is possible to provide information that the SGLT2 inhibitor has excellent drug responsiveness when the miR-136-3p, miR-15a-3p, miR-337-3p, miR-369-3p, miR-376a-3p, miR-379-5p or miR-495-3p expression is increased or the miR-150-3p, miR-483-3p or miR-6126 expression is decreased by administering the SGLT2 inhibitor.

Specifically, in a diabetes patient group after administering an antidiabetic compared to a diabetes patient group before administering the antidiabetic, when the miR-136-3p expression level is increased 2- to 3-fold, preferably 2.2- to 2.5-fold; the miR-150-3p expression level is decreased 1.5- to 2.5-fold, preferably 1.8- to 2.2-fold; the miR-15a-3p expression level is increased 1.5- to 2.5-fold, preferably 1.8- to 2.2-fold; the miR-337-3p expression level is increased 2- to 3-fold, preferably 2.2- to 2.5-fold; the miR-369-3p expression level is increased 1.5- to 2.5-fold, preferably 2- to 2.4-fold; the miR-376a-3p expression level is increased 2.5- to 3.5-fold, preferably 3-to 3.4-fold; the miR-379-5p expression level is increased 2.5- to 3.5-fold, preferably 2.6- to 3-fold; the miR-495-3p expression level is increased 2- to 3-fold, preferably 2.2- to 2.5-fold; the miR-483-3p expression level is decreased 1.5- to 2.5-fold, preferably 2- to 2.4-fold; or the miR-6126 expression level is decreased 1.5- to 2.5-fold, preferably 1.9- to 2.3-fold, it can be seen that there is an effect of treating diabetes by the drug.

In addition, after patient tissue-derived cells, preferably diabetes-related cells, are isolated before directly administering a drug to a patient according to the purpose, the isolated cells are treated with the drug, and then changes in microRNA expression levels may be confirmed to select an antidiabetic which is more suitable for patients and has excellent drug responsiveness.

Furthermore, the present invention, in yet another aspect, relates to a method for providing information on whether an antidiabetic is administered, the method including: (a) measuring the expression levels of one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126 in a biological sample derived from a diabetes patient; and
(b) comparing the microRNA expression level of the sample with the microRNA expression level of the normal control.

The antidiabetic may be a sodium glucose cotransporter 2 (SGLT2) inhibitor, and it is possible to provide information that an SGLT2 inhibitor is administered when the miR-136-3p, miR-15a-3p, miR-337-3p, miR-369-3p, miR-376a-3p, miR-379-5p or miR-495-3p expression is decreased, or the miR-150-3p, miR-483-3p or miR-6126 expression is increased in the diabetes patient compared to the normal control.

### [Advantageous Effects]

In the present invention, the drug responsiveness prediction markers miR-136-3p, miR-150-3p, miR-15a-3p, miR-337-3p, miR-369-3p, miR-376a-3p, miR-379-5p, miR-483-3p, miR-495-3p, and miR-6126 were selected by comparing miRNA profiles of a normal group and a diabetes patient group before and after administering an SGLT2 inhibitor, which is an antidiabetic. Although it was confirmed that the expression patterns of the selected markers differed between the normal group and the diabetes patient group, it was confirmed that the expression patterns for the diabetes patient group were restored so as to be similar to those for the normal group when the SGLT2 inhibitor, which is an antidiabetic, was administered to the diabetes patient group. Therefore, the marker according to the present invention can be useful as a marker for predicting drug responsiveness to antidiabetics.

### [Description of Drawings]

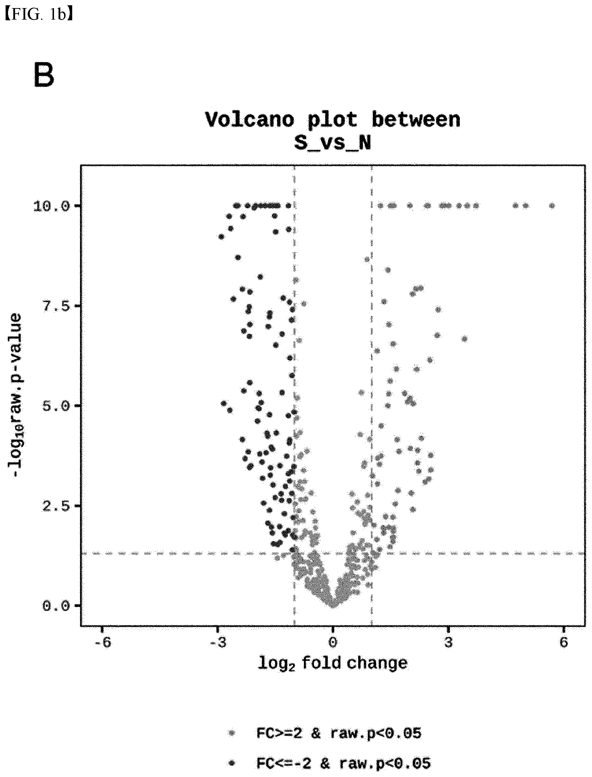

FIGS. 1A and 1B are comparative analyses of the expression levels of microRNAs in exosomes isolated from the sera of a normal group and a diabetes patient group before taking an SGLT2 inhibitor, (A) is a heatmap result of performing hierarchical clustering according to the expression level of microRNAs whose expression was significantly decreased or increased 2-fold or more in the diabetes patient group compared to the normal group, and (B) is a result of showing microRNA expression levels as a volcano plot in order to confirm the difference in expression levels between the normal group and the diabetes patient group before taking the SGLT2 inhibitor.

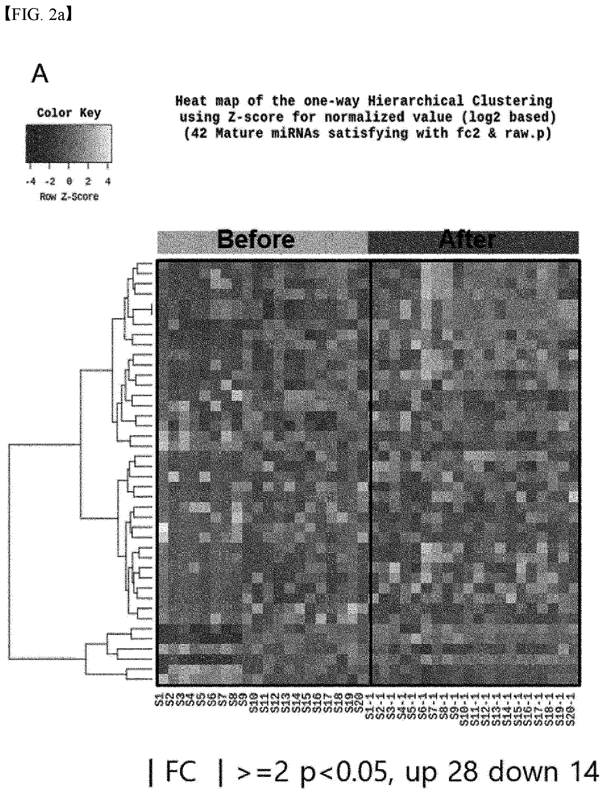

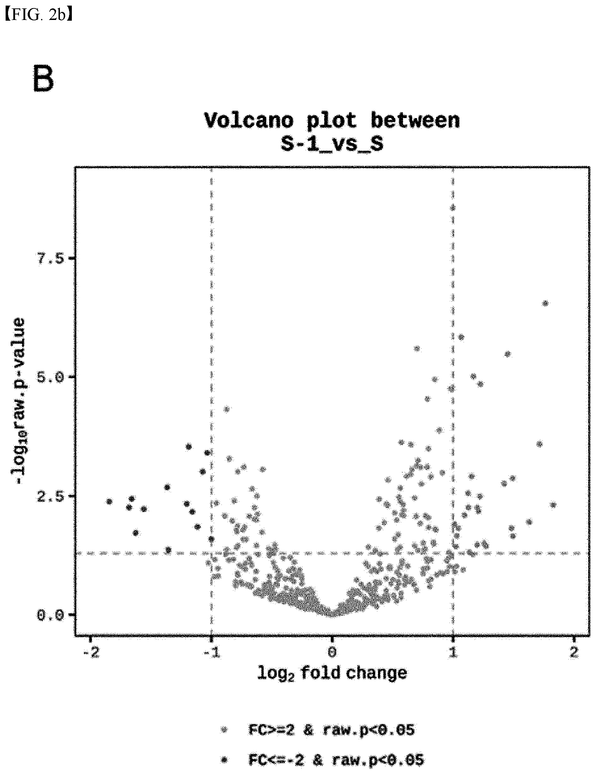

FIGS. 2A and 2B are comparative analyses of the expression levels of microRNAs in exosomes isolated from the sera of a diabetes patient group before taking an SGLT2 inhibitor and a diabetes patient group after taking an SGLT2 inhibitor, (A) is a heatmap result of performing hierarchical clustering according to the expression level of microRNAs whose expression was significantly decreased or increased 2-fold or more in the diabetes patient group after taking the SGLT2 inhibitor compared to the diabetes patient group before taking the SGLT2 inhibitor, and (B) is a result of showing microRNA expression levels as a volcano plot in order to confirm the difference in expression levels between the diabetes patient group before taking the SGLT2 inhibitor and the diabetes patient group after taking the SGLT2 inhibitor.

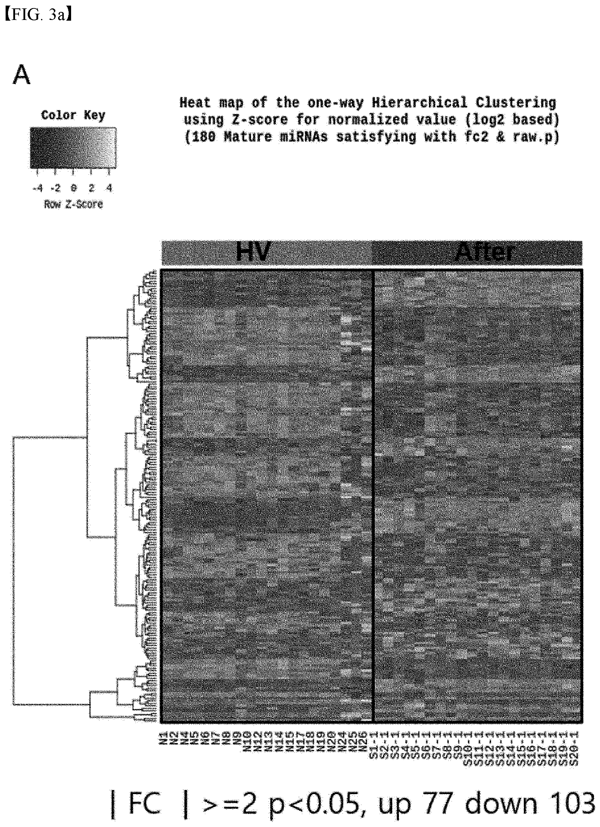

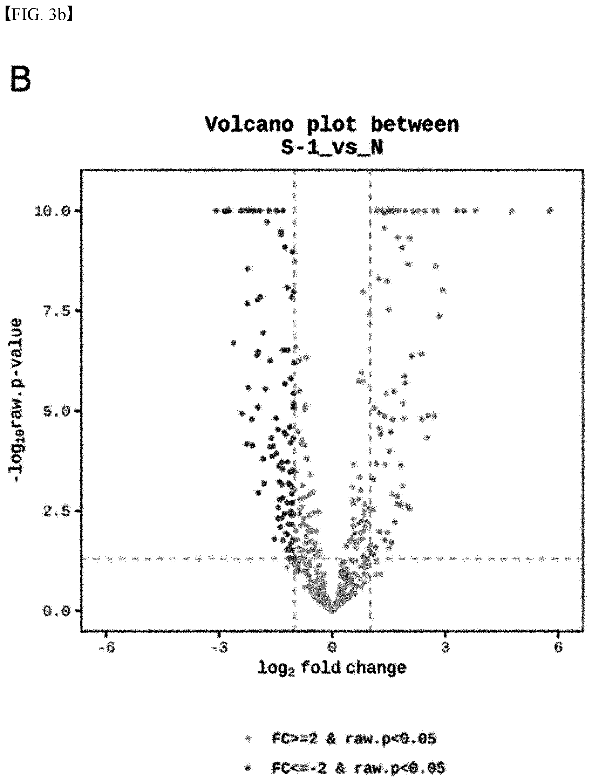

FIGS. 3A and 3B are comparative analyses of the expression levels of microRNAs in exosomes isolated from the sera of a normal group and a diabetes patient group after taking an SGLT2 inhibitor, (A) is a heatmap result of performing hierarchical clustering according to the expression level of microRNAs whose expression was significantly decreased or increased 2-fold or more in the diabetes patient group after taking the SGLT2 inhibitor compared to the normal group, and (B) is a result of showing microRNA expression levels as a volcano plot in order to confirm the difference in expression levels between the normal group and the diabetes patient group after taking the SGLT2 inhibitor.

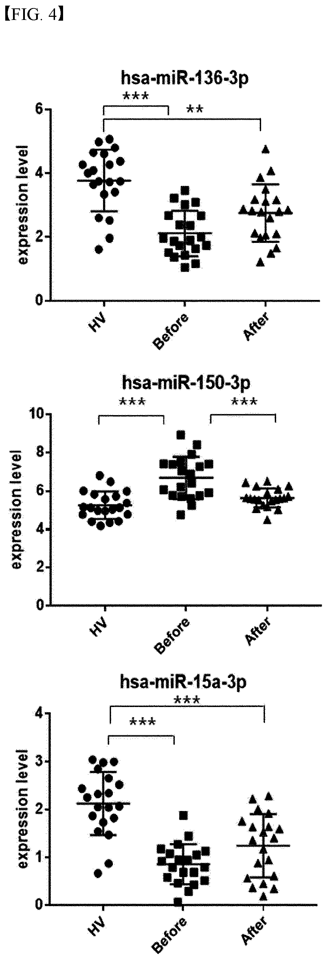

FIG. 4 illustrates the results of measuring and comparing the expression levels of miR-136-3p, miR-150-3p and miR-15a-3p among the normal group (HV), the diabetes patient group before taking the SGLT2 inhibitor (Before), and the diabetes patient group after taking the SGLT2 inhibitor (After).

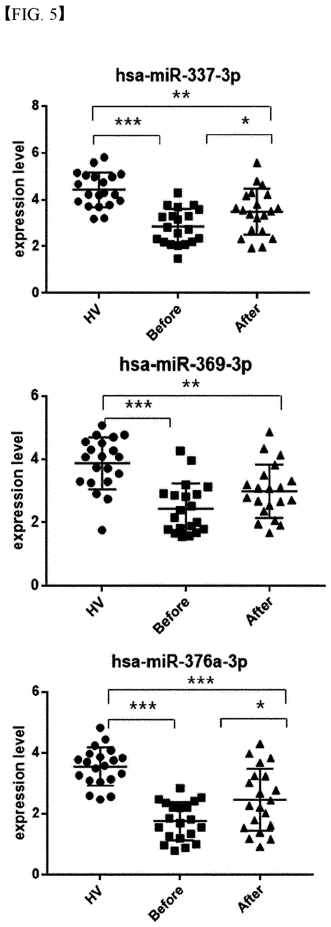

FIG. 5 illustrates the results of measuring and comparing the expression levels of miR-337-3p, miR-369-3p and miR-376a-3p among the normal group (HV), the diabetes patient group before taking the SGLT2 inhibitor (Before), and the diabetes patient group after taking the SGLT2 inhibitor (After).

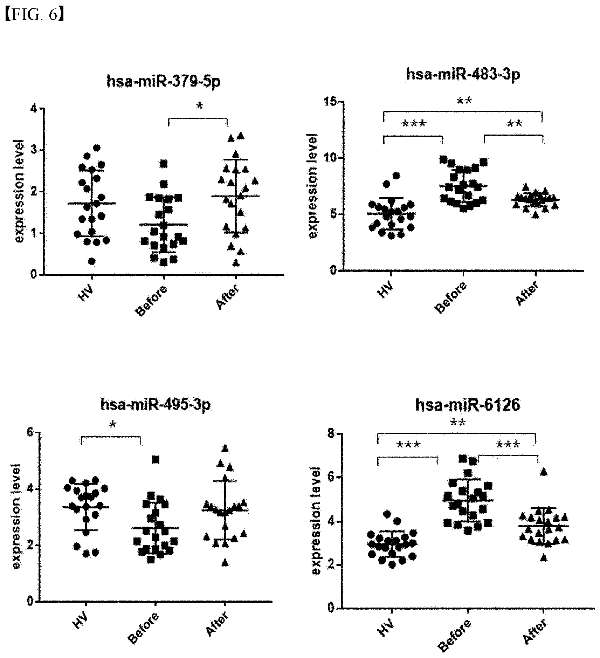

FIG. 6 illustrates the results of measuring and comparing the expression levels of miR-379-5p, miR-483-3p, miR-495-3p and miR-6126 among the normal group (HV), the diabetes patient group before taking the SGLT2 inhibitor (Before), and the diabetes patient group after taking the SGLT2 inhibitor (After).

### [Modes of the Invention]

### [Example 1]

### Patient selection and sequencing

### 1-1: Patient selection

In the present invention, in order to select biomarkers for predicting drug responsiveness to antidiabetics, 20 normal subjects, and 20 diabetes patients whose diabetic symptoms had been ameliorated by administering the SGLT2 inhibitor (SGLT2 inh-taking group) were selected from among diabetes patients who had never taken an SGLT2 inhibitor, and in the SGLT2 inh-taking group, serum samples were collected before and after taking the SGLT2 inhibitor, and analyzed.

The clinical characteristics of the patients are shown in the following Table 1, and the number in parentheses denotes the number of samples.

**[Table 1]**

| | Patient clinical information | | |
|---|---|---|---|
| | | Normal (20) | SLGT2 inh-taking group (20) |
| Gender (m/f) | | 0/20 | 12/8 |
| age | | 53.00±4.24 | 56.20±10.62 |
| height | | 157.68±5.55 | 164.43±9.84 |
| weight | | 54.26±6.00 | 73.09±10.62 |
| BMI | | 21.75±1.38 | 27.02±2.96 |
| waist | | 72.91±4.05 | 92.45±10.21 |
| FBS | | 86.90±3.73 | 147.05±30.89 |
| HbA1c | | 5.27±0.22 | 7.60±0.86 |
| WBC | | 5.74±1.29(10) | 6.89±1.49(19) |
| AST | | 23.05±5.37 | 24.89±10.59(19) |
| ALT | | 15.05±6.80 | 26.68±13.29(19) |
| BUN | | 14.38±3.70(8) | 13.68±3.77(19) |
| Creatinine | | 0.66±0.10 | 0.80±0.20(19) |
| LDL | | 116.96±30.48 | 113.60±40.39(18) |
| M/C ratio | | -(0) | 36.78±68.74(18) |
| eGFR | | 93.46±16.20(18) | 93.78±20.72(19) |
| HOMA-IR | | 0.32±0.36 | 3.09±3.30(15) |
| SBP | | 118.60±17.55 | 126.50±20.69 |
| DBP | | 75.15±7.81 | 80.05±16.70 |

### 1-2: RNA isolation from serum and sequencing

RNA was isolated from the sera of the normal group and the SLGT2 inh-taking group selected in Example 1-1, and analyzed, and in the SLGT2 inh-taking group, serum samples were collected before and after taking the SGLT2 inhibitor, and analyzed.

Specifically, exosomes were isolated, using Exoquick (SBI, USA), from serum samples collected from a normal group, a diabetes patient group before taking an SGLT2 inhibitor, and a diabetes patient group after taking an SGLT2 inhibitor, total RNA was extracted from the isolated exosomes using a Qiagen miRNeasy kit, and the quality of the extracted total RNA was measured using an Agilent RNA Bioanalyzer according to the manufacturer's instructions. Thereafter, using the isolated RNA, a library was prepared using a Takara SMARTer smRNA for an Illumina kit at Macrogen, Inc., and then, next-generation nucleotide sequencing (NGS) was performed using Hiseq2500 to detect microRNA.

### [Example 2]

### Selection of biomarkers for predicting drug responsiveness to antidiabetics

### 2-1: MicroRNA analysis of difference in expression level between normal group and diabetes patient group before taking SGLT2 inhibitor

First, in order to identify microRNAs (miRNAs) with a difference in expression level in the blood-derived exosomes of 20 subjects of the normal group and 20 subjects of the diabetes patient group before taking the SGLT2 inhibitor (miRNA), the present inventors isolated exosomes from serum collected from each subject according to the method of Example 1-2, and performed NGS analysis on the extracted total RNA. As a result of the analysis, 523 miRNAs were detected, and furthermore, the expression levels of the detected miRNAs were compared.

As a result, as illustrated in FIG. 1A, it was found that among the 523 miRNAs, there were 105 miRNAs whose expression was decreased 2-fold or more (p < 0.05) and 75 miRNAs whose expression increased 2-fold or more (p < 0.05) in the diabetes patient group before taking the SGLT2 inhibitor (Before) compared to the normal group (HV). In addition, FIG. 1B illustrates the volcano plot results according to the expression levels of miRNAs showing the aforementioned significant expression changes.

### 2-2: MicroRNA analysis of difference in expression level of diabetes patient group before taking SGLT2 inhibitor vs diabetic patient group after taking SGLT2 inhibitor

In order to identify miRNAs with a difference in expression level in the blood-derived exosomes of 20 subjects of the diabetes patient group before taking the SGLT2 inhibitor and 20 subjects of the diabetes patient group after taking the SGLT2 inhibitor (miRNA), the present inventors performed NGS analysis on total RNA in exosomes obtained by the same method as above. As a result of the analysis, 523 miRNAs were detected, and furthermore, the expression levels of the detected miRNAs were compared.

As a result, as illustrated in FIG. 2A, it was found that among the 523 miRNAs, there were 14 miRNAs whose expression was decreased 2-fold or more (p < 0.05) and 28 miRNAs whose expression increased 2-fold or more (p < 0.05) in the diabetes patient group after taking the SGLT2 inhibitor (After) compared to the diabetes patient group before taking the SGLT2 inhibitor (Before). In addition, FIG. 2B illustrates the volcano plot results according to the expression levels of miRNAs showing the aforementioned significant expression changes.

### 2-3: MicroRNA analysis of difference in expression level between normal group and diabetes patient group after taking SGLT2 inhibitor

In order to identify miRNAs with a difference in expression level in the blood-derived exosomes of 20 subjects of the normal group and 20 subjects of the diabetes patient group after taking the SGLT2 inhibitor (miRNA), the present inventors performed NGS analysis on total RNA in exosomes obtained by the same method as above. As a result of the analysis, 523 miRNAs were detected, and furthermore, the expression levels of the detected miRNAs were compared.

As a result, as illustrated in FIG. 3A, it was found that among the 523 miRNAs, there were 103 miRNAs whose expression was decreased 2-fold or more (p < 0.05) and 77 miRNAs whose expression increased 2-fold or more (p < 0.05) in the diabetes patient group after taking the SGLT2 inhibitor (After) compared to the normal group (HV). In addition, FIG. 3B illustrates the volcano plot results according to the expression levels of miRNAs showing the aforementioned significant expression changes.

### 2-4: Selection of biomarkers for predicting drug responsiveness

Referring to the results in FIGS. 1A to 3B, the expression levels differed between the normal group and the diabetes patient group before taking SGLT2 inhibitor, microRNAs whose expression pattern was restored to a level similar to that of the normal group by administering the SGLT2 inhibitor were finally selected, and selected markers and sequence information are shown in the following Tables 2 and 3.

**[Table 2]**

| | Fold change (+/-) | | | |
|---|---|---|---|---|
| Mature miRNA | Before/HV | After/Before | After/HV | ANOVA |
| hsa-miR-136-3p | -5.01 | 2.31 | -2.17 | ^{∗∗∗} |
| hsa-miR-150-3p | 2.37 | -2.05 | 1.16 | ^{∗∗∗} |
| hsa-miR-15a-3p | -4.48 | 2.06 | -2.18 | ^{∗∗∗} |
| hsa-miR-337-3p | -4.52 | 2.33 | -1.94 | ^{∗∗∗} |
| hsa-miR-369-3p | -4.62 | 2.18 | -2.12 | ^{∗∗∗} |
| hsa-miR-376a-3p | -7.48 | 3.28 | -2.28 | ^{∗∗∗} |
| hsa-miR-379-5p | -2.24 | 2.81 | 1.26 | ^{∗} |
| hsa-miR-483-3p | 4.23 | -2.17 | 1.95 | ^{∗∗∗} |
| hsa-miR-495-3p | -2.07 | 2.30 | 1.11 | ^{∗} |
| hsa-miR-6126 | 5.53 | -2.10 | 2.63 | ^{∗∗∗} |

**[Table 3]**

| | Marker sequence information | | |
|---|---|---|---|
| Mature miRNA | | Sequence | SEQ ID NO: |
| hsa-miR-136-3p | | CAUCAUCGUCUCAAAUGAGUC U | 1 |
| hsa-miR-150-3p | | CUGGUACAGGCCUGGGGAC AG | 2 |
| hsa-miR-15a-3p | | CAGGCCAUAUUGUGCUGCCUC A | 3 |
| hsa-miR-337-3p | | CUCCUAUAUGAUGCCUUUCUU C | 4 |
| hsa-miR-369-3p | | AAUAAUACAUGGUUGAUCUU U | 5 |
| hsa-miR-376a-3p | | AUCAUAGAGGAAAAUCCACG U | 6 |
| hsa-miR-379-5p | | UGGUAGACUAUGGAACGUAG G | 7 |
| hsa-miR-483-3p | | UCACUCCUCUCCUCCCGUCUU | 8 |
| hsa-miR-495-3p | | AAACAAACAUGGUGCACUUC UU | 9 |
| hsa-miR-6126 | | GUGAAGGCCCGGCGGAGA | 10 |

As shown in Table 2, miR-136-3p, miR-150-3p, miR-15a-3p, miR-337-3p, miR-369-3p, miR-376a-3p, miR-379-5p, miR -483-3p, miR-495-3p and miR-6126 markers were finally selected, and as a result of analyzing the expression level of each marker by dividing the samples into a normal group (HV), a diabetes patient group before taking an SGLT2 inhibitor (Before) and a diabetes patient group after taking an SGLT2 inhibitor, as illustrated in FIGS. 4 to 6, it was confirmed that in all the cases, a remarkable difference in expression level was observed between the normal group and the diabetes patient group before taking the antidiabetic, and the expression levels were restored so as to be similar to those for the normal group by taking the SGLT2 inhibitor.

### [Industrial Applicability]

In the present invention, the drug responsiveness prediction markers miR-136-3p, miR-150-3p, miR-15a-3p, miR-337-3p, miR-369-3p, miR-376a-3p, miR-379-5p, miR-483-3p, miR-495-3p, and miR-6126 were selected by comparing miRNA profiles of a normal group and a diabetes patient group before and after administering an SGLT2 inhibitor, which is an antidiabetic. Although it was confirmed that the expression patterns of the selected markers differed between the normal group and the diabetes patient group, it was confirmed that the expression patterns for the diabetes patient group were restored so as to be similar to those for the normal group when the SGLT2 inhibitor, which is an antidiabetic, was administered to the diabetes patient group. Therefore, the marker according to the present invention can be useful as a marker for predicting drug responsiveness to antidiabetics, and thus, has industrial applicability.

### [Sequence Listing Free Text]

SEQ ID NO: 1 represents the base sequence of a miR-136-3p marker.
SEQ ID NO: 2 represents the base sequence of a miR-150-3p marker.
SEQ ID NO: 3 represents the base sequence of a miR-15a-3p marker.
SEQ ID NO: 4 represents the base sequence of a miR-337-3p marker.
SEQ ID NO: 5 represents the base sequence of a miR-369-3p marker.
SEQ ID NO: 6 represents the base sequence of a miR-376a-3p marker.
SEQ ID NO: 7 represents the base sequence of a miR-379-5p marker.
SEQ ID NO: 8 represents the base sequence of a miR-483-3p marker.
SEQ ID NO: 9 represents the base sequence of a miR-495-3p marker.
SEQ ID NO: 10 represents the base sequence of a miR-6126 marker.

## Claims

1. A biomarker composition for predicting drug responsiveness to antidiabetics, comprising one or more microRNAs selected from the group consisting of miR-136-3p, miR-150-3p, miR-15a-3p, miR-337-3p, miR-369-3p, miR-376a-3p, miR-379-5p, miR-483-3p, miR-495-3p and miR-6126.

2. The biomarker composition of claim 1, wherein the miR-136-3p is represented by a base sequence of SEQ ID NO: 1, the miR-150-3p is represented by a base sequence of SEQ ID NO: 2, the miR-15a-3p is represented by a base sequence of SEQ ID NO: 3, the miR-337-3p is represented by a base sequence of SEQ ID NO: 4, the miR-369-3p is represented by a base sequence of SEQ ID NO: 5, the miR-376a-3p is represented by a base sequence of SEQ ID NO: 6, the miR-379-5p is represented by a base sequence of SEQ ID NO: 7, the miR-483-3p is represented by a base sequence of SEQ ID NO: 8, the miR-495-3p is represented by a base sequence of SEQ ID NO: 9, and the miR-6126 is represented by a base sequence of SEQ ID NO: 10.

3. The biomarker composition of claim 1, wherein the antidiabetic is a sodium glucose cotransporter 2 (SGLT2) inhibitor.

4. The biomarker composition of claim 1, wherein the diabetes is type 2 diabetes.

5. A composition for predicting drug responsiveness to antidiabetics, comprising an agent that measures the expression level of the biomarker composition of claim 1.

6. The composition of claim 5, wherein the agent that measures the expression level is a sense and antisense primer or probe, which complementarily binds to microRNA.

7. A diagnostic kit for predicting drug responsiveness to antidiabetics, comprising an agent that measures the expression level of the biomarker composition of claim 1.

8. A method for providing information on drug responsiveness to an antidiabetic, the method comprising: (a) measuring the expression level of the biomarker composition of claim 1 in a biological sample derived from a subject before and after the administration of the antidiabetic; and
(b) comparing the microRNA expression levels before and after the administration of the antidiabetic.

9. The method of claim 8, wherein the expression level in Step (a) is measured through one or more methods selected from the group consisting of next generation sequencing (NGS), polymerization chain reaction (PCR), reverse transcription-polymerase chain reaction (RT-PCR), real-time polymerization chain reaction (PCR), an RNase protection assay (RPA), a microarray, and northern blotting.

10. The method of claim 8, wherein after the administration of the antidiabetic, when the miR-136-3p, miR-15a-3p, miR-337-3p, miR-369-3p, miR-376a-3p, miR-379-5p or miR-495-3p expression is increased or the miR-150-3p, miR-483-3p or miR-6126 expression is decreased, the drug responsiveness to the antidiabetic is excellent, so that information that the antidiabetic is effective for treating diabetes is provided.
